# EUROPEAN PATENT SPECIFICATION

(11) **EP 0 308 040 B1**
(45) Date of publication and mention of the grant of the patent: **20.04.1994**
(21) Application number: 88305933.9
(22) Date of filing: 30.06.1988
(51) Int. Cl.: A61M 5/00

(54) **Single use disposable syringe**
Einmalverwendbare Wegwerfspritze
Seringue jetable à usage unique

(30) Priority: 18.09.1987 WO PCT/US87/02408
(43) Date of publication of application: 22.03.1989
(73) Proprietor: PATH, Seattle, WA 98109 (US)
(72) Inventor: Free, Michael J., Seattle Washington 98115 (US); Ellard, Terence R., Seattle, Washington 98103 (US)
(74) Representative: Smith, Norman Ian

(56) References cited:
- DE-A- 1 070 785
- DE-A- 3 107 414
- FR-A- 1 257 067
- FR-A- 2 319 383
- US-A- 2 216 354
- US-A- 2 618 263
- US-A- 4 391 273

## Description

### Technical Field

This invention relates to a single use disposable syringe, and more particularly, to an essentially rigid syringe having a fixed needle wherein the syringe is rendered unusable by including a hard flute or disc which through interaction of relative moving parts only permits a single cycle of the plunger before locking it in position.

### Background Art

Standard disposable syringes, although intended for one time use, are constructed in such a way that they can be re-used. Proper one-time use, destruction and disposal of these devices requires strict compliance on the part of the user, generally in a controlled environment.

In an environment which does not include appropriate controls, i.e. outside of a hospital or clinic, disposable syringes have gotten into the hands of people who have used them improperly; e.g. employing them for injections of illegal drugs, or injection into two or more persons, without adequate sterilization between uses.

In addition to the diversion of used disposable syringes from legitimate sources for administration of illegal and dangerous drugs, the syringes, when not properly sterilized, become a vehicle for the transmission of diseases including the HIV virus (AIDS) and hepatitis B.

One of the methods proposed to prevent the reuse of syringes has been to package the medication in a single dose self-destructive collapsible container which is then squeezed and collapsed to administer the medication.

However, not all medications are suitable for use in prefilled single dose systems. Some medications are most economically and desirably distributed in a multi-dose container. These can then be administered with a single use, disposable syringe, or a multiple use sterilizable syringe.

Prior art devices which have dealt with the problem of reusable syringes in one form or another include U. S. Patent No. 2,618,263 granted to Lakso et al November 18, 1952 which discloses a premeasured medication in an ampule adjacent to the inner end of a needle. Upon pressure being applied to the ampule, the needle penetrates the ampule and the medication is discharged. The entire device is then disposed.

U. S. Patent No. 4,013,073 granted to Cunningham on March 22, 1977 discloses a collapsible single use syringe wherein the interior of the collapsible wall is constructed such that when the walls are pressed together to discharge the medication they interlock and therefore render the device impossible to reuse.

U. S. Patent No. 4,022,206 granted to Hilleman et al May 10, 1977 discloses a method and apparatus for storing and delivering a lyophilized vaccine in a single dose prepackaged system but makes no provision for rendering the unit not reusable.

U. S. Patent No. 4,391,273 granted to Chiquiar-Arias July 5, 1983 discloses a rigid type syringe including a pin which is attached to the piston and which penetrates the bottom wall of the cylinder when the injection has been completed. In the alternative, this patent discloses a knife which permits movement of the cylinder in a forward direction but cuts the sidewall of the cylinder if there is an attempt to recycle the piston or reuse the syringe.

U.S. Patent No. 4,548,601 granted to Larry on October 22, 1985 discloses a prepackaged pharmaceutical combination including an inner and outer flexible envelope such that the inner envelope will remain collapsed due to atmospheric pressure once the injection is completed.

DE-A-3107414 describes a single use syringe having a cylinder and a plunger. The plunger carries a disc which engages a shoulder on the cylinder and acts to prevent retraction of the plunger after it has been operated.

According to a first aspect of the present invention there is provided a single use syringe comprising: an elongate hollow cylindrical barrel having an interior sidewall, a proximal end, a distal end, and a nozzle closing said distal end of said barrel;
a plunger within said barrel for axial movement therein for drawing fluid into and expelling fluid out of said barrel through said nozzle, said plunger member comprising a plunger shaft extending beyond said barrel at said proximal end and having a piston at the distal end;
a control device locatable between the plunger shaft and the interior wall at a position which determines the volume of the syringe to be filled with medication, characterised in that the control device comprises a flute-like element having legs, which are flared outwardly and sharpened at the upper end, and which include a spur-like portion of a material harder than the interior side wall and contacting and angled against the interior side wall so that the control device is free to move towards the distal end but proximal movement of the plunger shaft after the piston contacts the distal portion of the control device, causes the spur-like element to embed into the interior side wall to resist further proximal movement of the piston and control device, and a chisel-like portion contacting and biased against the plunger shaft so that the plunger shaft is free to move a first time toward the proximal end but distal movement of the plunger shaft causes the chisel-like portion to engage the plunger shaft so that the control device moves with the plunger shaft toward the distal end, the arrangement being such that the control device permits the syringe to be loaded with medication in the volume defined by the control device by proximal movement of the piston as far as the distal end of the control device, and after depression of the plunger shaft to expel the medication, any second proximal movement of the plunger shaft is prevented by the spur-like portion embedding into the interior side wall.

According to a second aspect of the present invention there is provided a single use syringe comprising: an elongate hollow cylindrical barrel having an interior sidewall, a proximal end, a distal end, and a nozzle closing said distal end of said barrel;
a plunger within said barrel for axial movement therein for drawing fluid into and expelling fluid out of said barrel through said nozzle, said plunger member comprising a plunger shaft extending beyond said barrel at said proximal end and having a piston at the distal end, a control device locatable between the plunger shaft and the interior wall at a position which determines the volume of the syringe to be filled with medication, characterised in that the control device is formed from bent wire which has the shape of a distorted U-configuration and comprises a pair of upwardly and outwardly flared arm members each of which is sharpened to a chisel-like point so that when placed in position between the interior sidewall of the cylindrical barrel and the plunger the plunger shaft is free to move a first time toward the proximal end but distal movement of the plunger shaft causes the chisel-like portion to engage the plunger shaft so that the control device moves with the plunger shaft toward the distal end, the arrangement being such that the control device permits the syringe to be loaded with medication in the volume defined by the control device by proximal movement of the piston as far as the distal end of the control device, and after depression of the plunger shaft to expel the medication, any second proximal movement of the plunger shaft is prevented by the spur-like portion embedding into the interior sidewall.

The invention will be described now by way of example only, with particular reference to the accompanying drawings. In the drawings:

Figure 1 is a pictorial representation of a typical syringe which would utilize the present invention.

Figure 2 is an isometric view of the plunger/cylinder assembly.

Figure 3 is a view of a syringe in accordance with the present invention with a flute in place within the syringe.

Figure 4 is a vertical view of the flute of Figure 3 outside the syringe.

Figure 5 is a bottom plan view of the flute of Figure 3.

Figure 6 is an isometric of the flute of Figures 3 to 5 in its operative position.

Figure 7 is a sectional view along line 3-3 of Figure 1 depicting the flute in position prior to use of the syringe.

Figure 8 is similar to Figure 7 showing the syringe with the measuring tube filled with medicine.

Figure 9 is a depiction similar to that of Figure 7 showing the apparatus following the administration of the medication and with the cylinder locked against the end of the measuring tube.

Figure 10 is a representation of another embodiment of the present invention.

Figure 11 is a view along line 13-13 of Figure 10.

Figure 12 is a side elevation view of the device of Figure 10.

### Best Mode For Carrying Out The Invention

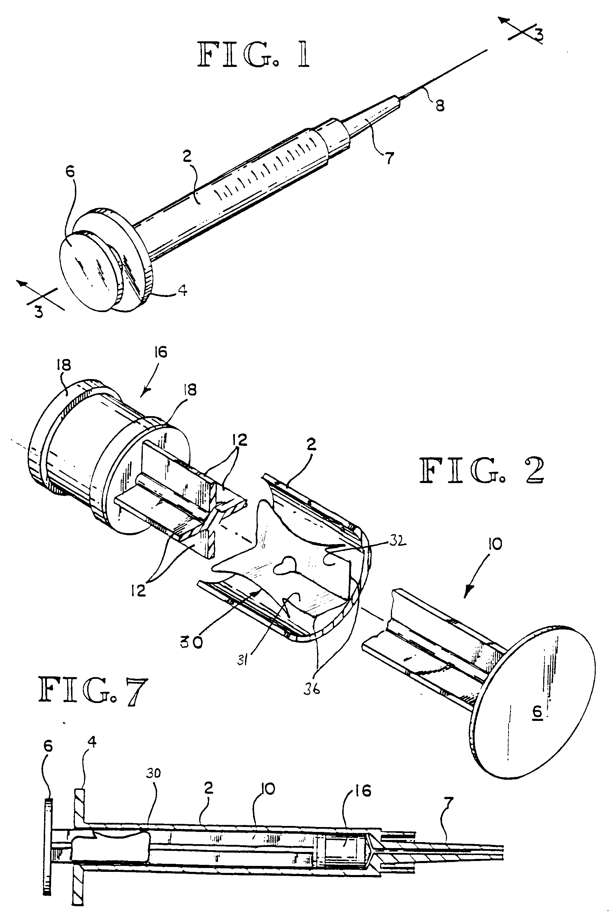

As seen in Figure 1, the environment of the present invention is a rigid plastic syringe having a main cylinder 2, an outwardly extending upper disk portion 4 for ease in administering the medication, a cap 6 which is the terminous of the outer end of the plunger as described hereinafter and an administering element 8 protruding from noes 7.

Referring now to Figure 2, the cap member 6 as seen in the lower end of this figure is connected to or integral with plunger member 10. Plunger member 10 is generally of X configuration in cross-section having legs 12 and a central interconnecting cylindrical member 14. Piston member 16, including sealing elements 18, completes the plunger element at the opposite end to cap 6. Approximately midway of the plunger member, in this figure, there is depicted a portion of the cylinder 2 with a flute 30.

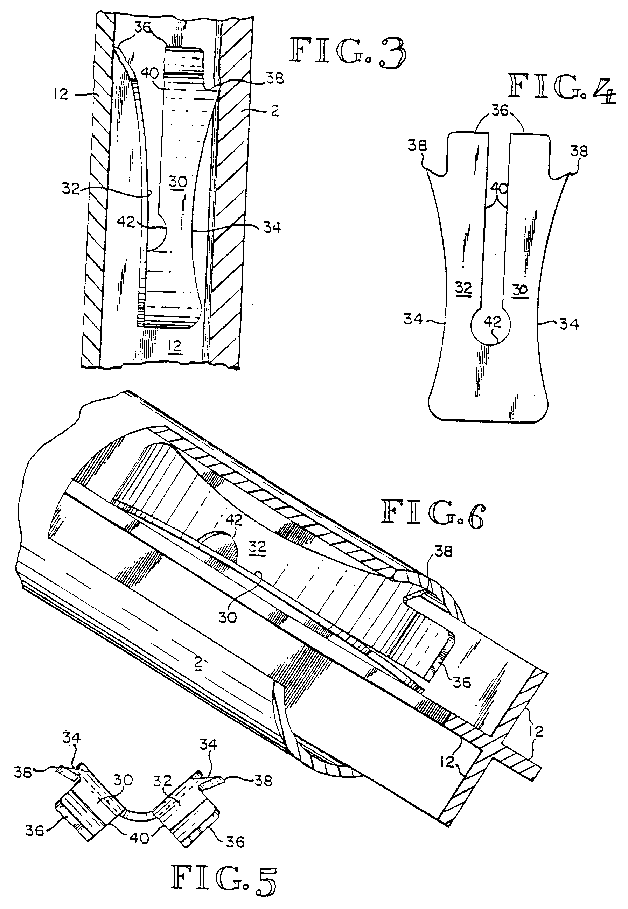

Referring to Figure 4 the thin metal flute includes legs 30, 32 having a slight waist portion 34. At the upper portion, the legs are separated by a slot 40 terminating in the lower portion with a stress reducing arcuate portion 42.

Figure 5 shows the flute in end view in which legs 30 and 32 can be seen to be disposed at right angles to each other, flared outwardly and sharpened at the upper end including a chisel portion 36 and a pointed dog 38.

It is to be understood that the internal elements 36 which are in contact with the plunger shaft 10 are arranged such that movement relative to the two contigous elements in one direction is substantially without resistance whereas movement in the opposite direction causes the elements 36 to embed themselves in the shaft. Likewise, the ouwardly projecting portion 36 will readily move in one direction along the inside wall of cylinder 2 but any attempt at relative movement in the opposite direction will cause the portion 26 to impinge in the inner wall of cylinder 2.

It is to be understood that the most consistent operation will be generated by utilizing a material for the flute, such as stainless steel which has memory and is hard, while utilizing a plastic material for the syringe itself. The relative hardness between the two materials permits the flute 30 to react against relative movement of contiguous elements, impinging itself into the softer material, and preventing further movement.

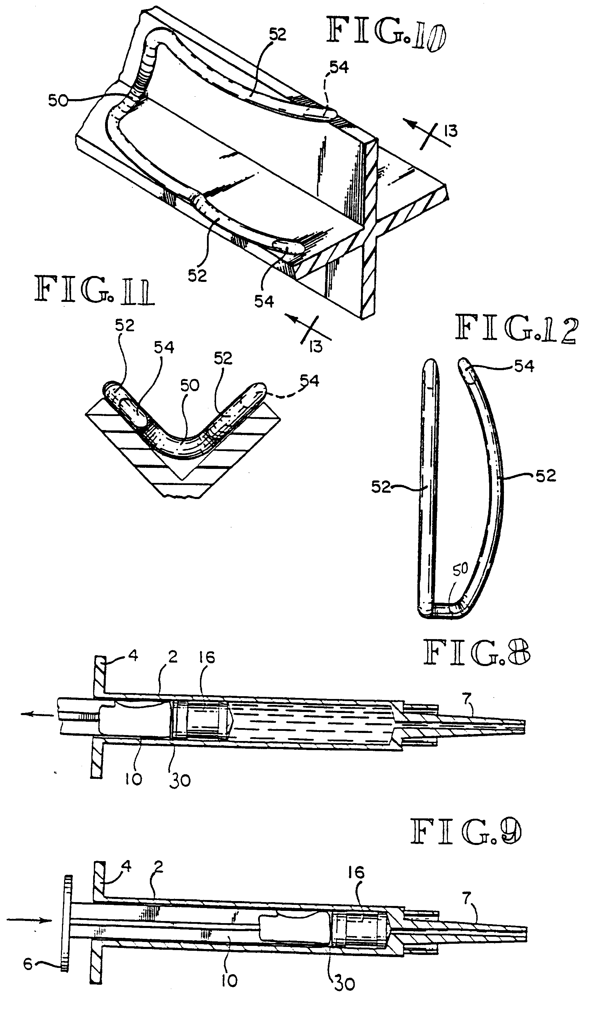

Referring now to Figure 7, the device is shown in cross-section without the needle in a configuration wherein the syringe is empty and ready for use. It is to be noted that the piston member 16 is adjacent the nose 7 and the flute 30 is located a predetermined distance to the rear of the cavity such that a withdrawal of the cylinder to this point will draw a predetermined amount of medication into the cylinder for administration. The ring is prevented from moving rearwardly because the darts 26 are locked to the inner wall of the cylinder. The plunger, however, is free to move in this direction.

It is to be noted that the flute 30 can readily be used, depending upon initial placement, to control the syringe volume.

Figure 8 depicts the syringe with the piston member 16 withdrawn to a position adjacent the flute 30, the cylinder being filled with the medication and the cylinder prevented from further rearward movement by the flute 30, the portions of which impinge on the inner wall of the cylinder.

As seen in Figure 9, the medication has been applied, the forward movement of plunger 10 has carried the flute 30 to its forwardmost position locking the piston 16 against the front of the cylinder 2. The flute was moved forward through coaction with the plunger. Any attempted rearward movement of the plunger will again cause the portion 38 on the flute 30 to impinge in the inner wall of the cylinder 2 preventing further reverse movement.

Thus in the present embodiment the restrictive flute is placed in position between the interior sidewall of the cylindrical portion of the syringe 2 and the legs 12 of the plunger such that a chisel portion 36 will dig into the plunger legs 12 when there is relative movement therebetween in one direction whereas relative movement in the other direction is substantially without resistance. The spur 38 will impinge itself in the side wall of the cylinder 2 when there is relative movement therebetween in one direction whereas relative movement in the other direction is substantially without resistance. The plunger can thus be withdrawn, drawing fluid into the cylinder until the piston 16 in Figure 2 is prevented from further outward movement by the flute impinged in the side wall of the cylinder 2. During the injection process, the chisel portion of the flute 36 will impinge on the legs 12 of the plunger and will be moved downwardly with the plunger shaft until the fluid is ejected. The flute then prevents rearward movement of the plunger by means of the spur 38 which will again embed into the sidewall of the cylinder 2.

The present embodiment allows for easy assembly into standard disposable syringes either at the time of manufacture or later, and will accomplish the purpose of allowing the plunger to be retracted to draw the fluid into the syringe up to a predetermined volume and then allow the plunger to expel the fluid and will also prevent a second retraction.

Referring now to Figure 10, another embodiment of the present invention is shown. This embodiment comprises a wire form which is generally in the shape of a distorted U-configuration having a base member 50 which is bent to generally conform to the V-formed in the sidewall of the plunger and a pair of upwardly and outwardly flared arm members 52 each of which is sharpened to a chisel-type point 54 such that when placed in position between the interior sidewall of the cylinder and the plunger for the piston it acts similarly to the restrictive flute device as described with reference to Figures 1 to 9.

Thus as can be seen, the present invention contemplates an inexpensive method of changing a standard disposable syringe to one that is only useable once. Further, use of the present invention allows a syringe of a given size to be used restrictively to administer differing volumes of medication by varying the placement of the device.

## Claims

1. A single use syringe comprising: an elongate hollow cylindrical barrel (2) having an interior sidewall, a proximal end, a distal end, and a nozzle (8) closing said distal end of said barrel;
a plunger (10) within said barrel for axial movement therein for drawing fluid into and expelling fluid out of said barrel through said nozzle, said plunger member comprising a plunger shaft extending beyond said barrel at said proximal end and having a piston (16) at the distal end;
a control device (30,32) locatable between the plunger shaft and the interior wall at a position which determines the volume of the syringe to be filled with medication, characterised in that the control device comprises a flute-like element having legs (30, 32), which are flared outwardly and sharpened at the upper end, and which include a spur-like portion (38) of a material harder than the interior side wall and contacting and angled against the interior side wall so that the control device is free to move towards the distal end but proximal movement of the plunger shaft after the piston contacts the distal portion of the control device, causes the spur-like element to embed into the interior side wall to resist further proximal movement of the piston and control device, and a chisel-like portion (36) contacting and biased against the plunger shaft so that the plunger shaft is free to move a first time toward the proximal end but distal movement of the plunger shaft causes the chisel-like portion to engage the plunger shaft so that the control device moves with the plunger shaft toward the distal end, the arrangement being such that the control device (30,32) permits the syringe to be loaded with medication in the volume defined by the control device by proximal movement of the piston as far as the distal end of the control device, and after depression of the plunger shaft to expel the medication, any second proximal movement of the plunger shaft is prevented by the spur-like portion embedding into the interior side wall.

2. A single use syringe comprising: an elongate hollow cylindrical barrel (2) having an interior sidewall, a proximal end, a distal end, and a nozzle (8) closing said distal end of said barrel;
a plunger (10) within said barrel for axial movement therein for drawing fluid into and expelling fluid out of said barrel through said nozzle, said plunger member comprising a plunger shaft extending beyond said barrel at said proximal end and having a piston (16) at the distal end, a control device (30,32) locatable between the plunger shaft and the interior wall at a position which determines the volume of the syringe to be filled with medication, characterised in that the control device is formed from bent wire which has the shape of a distorted U-configuration and comprises a pair of upwardly and outwardly flared arm members (52) each of which is sharpened to a chisel-like point (54) so that when placed in position between the interior sidewall of the cylindrical barrel (2) and the plunger (20) the plunger shaft is free to move a first time toward the proximal end but distal movement of the plunger shaft causes the chisel-like portion to engage the plunger shaft so that the control device moves with the plunger shaft toward the distal end, the arrangement being such that the control device (30,32) permits the syringe to be loaded with medication in the volume defined by the control device by proximal movement of the piston as far as the distal end of the control device, and after depression of the plunger shaft to expel the medication, any second proximal movement of the plunger shaft is prevented by the spur-like portion embedding into the interior sidewall.

3. A syringe according to claim 1 or claim 2, wherein said plunger shaft includes longitudinally oriented planar legs (12) having surfaces for engaging said chisel-like portion (36, 54).

4. A syringe according to claim 1, wherein said control device includes a second spur-like portion (38) and a second chisel-like portion (34).

5. A syringe according to claim 4, wherein said chisel-like portion and said second chisel-like portion are separated by a longitudinal slot (40) in said element so that said chisel-like portion and said second chisel-like portion extend proximally in cantilever spring-like fashion from said distal portion of said device forcing said chisel-like portion and said second chisel-like portion against said interior side wall of said barrel.

6. A syringe according to claim 4, wherein said plunger shaft includes at least two longitudinally oriented planar legs (12) having surfaces for engaging said chisel-like portion (36) and said second chisel-like portion (36) said legs (12) being positioned so that said chisel-like portions engage different planar legs (12).

7. A syringe according to claim 6, wherein at least two of said planar legs (12) are oriented substantially perpendicularly with respect to each other forming a V-shaped axial groove along the length of said plunger shaft for engaging said control device.

8. A syringe according to any preceding claim, wherein said barrel (2) is made of plastic.

9. A syringe according to any preceding claim, wherein said control device (30, 32) is made of metal.

10. A syringe according to claim 8, wherein said control device (30, 32) is made of sheet metal.

11. A syringe according to claim 9, wherein said sheet metal is stainless steel.

12. A syringe according to claim 1, wherein said plunger shaft has a plurality of longitudinally planar legs (12) and wherein said control device is placed only between adjacent planar legs of said plunger shaft.

## Patentansprüche

1. Eine einmal verwendbare Spritze wird bereitgestellt, umfassend: ein längliches, hohles zylindrisches Rohr (2), das eine innere Seitenwand, ein nahes Ende, ein fernes Ende und eine Düse (8) hat, die das genann ferne Ende des genannten Rohres schließt;
ein Kolbenelement (10), in dem genannten Rohr zur axialen Bewegung darin, um Fluid in das genannte Rohr durch die genannte Düse einzuziehen und aus diesem auszustoßen, wobei das genannte Kolbenelement einen Kolben schafft, umfaßt, der sich über das genannte Rohr an seinem nahen Ende hinaus erstreckt, und an seinem fernen Ende einen Kolben (60) hat;
eine Steuereinrichtung (30, 32), die zwischen dem Kolbenschaft und der Innenwand an einer Stelle anzuordnen ist, die das Volumen der Spritze bestimmt, die mit Medikament gefüllt werden soll, **dadurch gekennzeichnet**, daß die Steuereinrichtung ein hohlkantprofilförmiges Element umfaßt, das Arme (30, 32) hat, die nach außen gespreizt und an dem oberen Ende geschärft sind und die einen spitzenförmigen Abschnitt (38) aus einem Material einschließen, das härter als die innere Seitenwand ist, und die innere Seitenwand berühren und gegen sie gewinkelt sind, so daß die Steuereinrichtung frei ist, sich in Richtung zu dem fernen Ende zu bewegen, wobei aber eine Bewegung des Kolbenschaftes zu dem nahen Ende, nachdem der Kolben den fernen Abschnitt der Steuereinrichtung berührt, bewirkt, daß sich das spitzenförmige Element in die innere Seitenwand einbettet, um einer weiteren Bewegung des Kolbens zu dem nahen Ende und der Steuereinrichtung zu widerstehen, und einen messerförmigen Abschnitt (36), dar den Kolbenschaft berührt und gegen ihn vorbelastet ist, so daß sich der Kolbenschaft frei ein erstes Mal in Richtung zu dem nahen Ende bewegen kann, aber eine Bewegung des Kolbenschaftes zu dem fernen Ende bewirkt, daß der messerförmige Abschnitt an dem Kolbenschaft eingreift, so daß sich die Steuereinrichtung mit dem Kolbenschaft in Richtung zu dem fernen Ende bewegt, wobei die Ausgestaltung derart ist, daß die Steuereinrichtung (30, 32) gestattet, daß die Spritze mit Medikament in da Volumen geladen werden kann, das durch die Steuereinrichtung durch Bewegung des Kolbens zum nahen Ende soweit wie das ferne Ende der Steuereinrichtung festgelegt ist, und nach Eindrücken des Kolbenschaftes, um das Medikament auszustoßen, irgendeine zweite Bewegung des Kolbenschaftes zum nahen Ende durch den spitzenförmigen Abschnitt verhindert wird, der in die innere Seitenwand eindringt.

2. Eine einmal verwendbare Spritze umfassend: ein längliches, hohles, zylindrisches Rohr (2), das eine innere Seitenwand, ein nahes Ende, ein fernes Ende und eine Düse (8) hat, die das ferne Ende des genannten Rohres schließt;
ein Kolbenelement (10) innerhalb des Rohres zur axialen Bewegung darin, um Fluid in das genannte Rohr durch die genannte Düse einzuziehen und aus ihm auszustoßen, wobei das genannte Kolbenelement umfaßt, einen Kolbenschafft, der sich über das genannte Rohr an dem genannten nahen Ende hinauserstreckt und einen Kolben (16) an dem fernen Ende hat, eine Steuereinrichtung (30, 32), die zwischen dem Kolbenschaft und der Innenwand an einer Position anzuordnen ist, die das Volumen der mit Medikament zu füllenden Spritze bestimmt, **dadurch gekennzeichnet**, daß die Steuereinrichtung aus einem gebogenen Draht gebildet ist, der die Form einer verbogenen U-Konfiguration hat und ein Paar von sich nach oben und nach außen erweiternden Armgliedern (52) umfaßt, von denen jedes zu einer messerförmigen Spitze (54) so geschärft ist, daß, wenn es in seiner Lage zwischen der inneren Seitenwand des zylindrischen Rohres (2) und dem Kolben (20) angeordnet ist, der Kolbenschaft frei ist, sich ein erstes Mal in Richtung zu dem nahen Ende zu bewogen, aber eine Bewegung des Kolbenschaftes zu dem fernen Ende bewirkt, daß der messerförmige Abschnitt an dem Kolbenschaft so eingreift, daß sich die Steuereinrichtung mit dem Kolbenschaft in Richtung zu dem fernen Ende bewegt, wobei die Ausgestaltung derart ist, daß die Steuereinrichtung (30, 32) gestattet, daß die Spritze mit Medikament in dem Volumen, das durch die Steuereinrichtung begrenzt ist, durch eine Bewegung des Kolbens zum nahen Ende so weit wie das ferne Ende der Steuereinrichtung zu laden, und nach dem Niederdrücken des Kolbenschaftes das Medikament auszustoßen, wobei irgendeine zweite Bewegung des Kolbenschaftes zu dem nahen Ende durch den spitzenförmigen Abschnitt verhindert wird, der in die innere Seitenwand eindringt.

3. Eine Spritze gemäß Anspruch 1 oder Anspruch 2, bei der der genannte Kolbenschaft in Längsrichtung ausgerichtete, ebene Arme (12) einschließt, die Oberflächen zum Eingreifen des genannten messerförmigen Abschnittes (36, 54) haben.

4. Eine Spritze gemäß Anspruch 1, bei der die genannte Steuereinrichtung einen zweiten spitzenförmigen Abschnitt (38) und einen zweiten messerförmigen Abschnitt (34) einschließt.

5. Eine Spritze gemäß Anspruch 4, bei der der genannte messerförmige Abschnitt und der genannte zweite messerförmige Abschnitt durch einen Längsschlitz (40) in dem genannten Element so getrennt sind, daß sich der genannte messerförmige Abschnitt und der genannte zweite, messerförmige Abschnitt zum nahen Ende in freitragender, federähnlicher Form von dem genannten fernen Abschnitt der genannten Einrichtung erstrecken, wobei der genannte messerförmige Abschnitt und der genannte zweite, messerförmige Abschnitt gegen die genannte Innenseitenwand des genannten Rohres gedrückt werden.

6. Eine Spritze gemäß Anspruch 4, bei der der genannte Kolbenschaft wenigstens zwei in Längsrichtung ausgerichtete, ebene Arme (12) einschließt, die Oberflächen zum Eingreifen des genannten messerförmigen Abschnittes (36) und des genannten zweiten, messerförmigen Abschnittes (36) haben, wobei die Arme (12) so positioniert sind, daß die genannten messerförmigen Abschnitte an unterschiedlichen, ebenen Armen (12) angreifen.

7. Eine Spritze gemäß Anspruch 6, bei der wenigstens zwei der genannten ebenen Arme (12) im wesentlichen senkrecht in bezug zueinander ausgerichtet sind, wobei sie eine V-förmige, axiale Nut in Längsrichtung des genannten Kolbenschaftes zum Eingreifen der genannten Steuereinrichtung bilden.

8. Eine Spritze gemäß irgendeinem vorhergehenden Anspruch, bei der das genannte Rohr (2) aus Kunststoff hergestellt ist.

9. Eine Spritze gemäß irgendeinem vorhergehenden Anspruch, bei der die genannte Steuereinrichtung (30, 32) aus Metall hergestellt ist.

10. Eine Spritze gemäß Anspruch 8, bei der die genannte Steuereinrichtung (30, 32) aus einem Metallblech hergestellt ist.

11. Eine Spritze gemäß Anspruch 9, bei der das genannte tallblech rostfreier Stahl ist.

12. Eine Spritze gemäß Anspruch 1, bei der der genannte Kolbenschaft eine Mehrzahl länglicher, ebener Arme (12) hat und bei der die genannte Steuereinrichtung nur zwischen benachbarten, ebenen Armen des genannten Kolbenschaftes angeordnet ist.

## Revendications

1. Une seringue à usage unique comprenant : un corps cylindrique creux allongé (2) possédant une paroi latérale intérieure, une extrémité proximale, une extrémité distale et une buse (8) fermant ladite extrémité distale dudit corps ;
un poussoir (10) disposé à l'intérieur dudit corps pour s'y déplacer axialement afin d'aspirer du fluide dans ledit corps et de l'en expulser à travers ladite buse, ledit élément formant poussoir comprenant une tige de poussoir qui s'étend au-delà dudit corps au niveau de ladite extrémité proximale, et possédant un piston (16) à l'extrémité distale ;
un dispositif de blocage (30, 32) pouvant être disposé entre la tige de poussoir et la paroi intérieure, dans une position qui détermine le volume de la seringue que l'on veut remplir de médicament, caractérisée en ce que le dispositif de blocage comprend un élément en forme de cannelure comportant des pattes (30, 32) qui sont évasées vers l'extérieur et aiguisées à leur extrémité supérieure et qui possèdent une partie en forme d'ergot (38) qui est réalisée dans un matériau plus dur que la paroi latérale intérieure et qui est en contact, selon un angle, avec la paroi latérale intérieure, de façon que le dispositif de blocage puisse se déplacer librement en direction de l'extrémité distale, mais que le déplacement proximal de la tige de poussoir, une fois que le piston a touché la partie distale du dispositif de blocage, ait pour effet d'enfoncer l'élément en forme d'ergot dans la paroi latérale intérieure pour interdire tout nouveau déplacement proximal du piston et du dispositif de blocage, et une partie analogue à un burin (36) qui est en contact, sous l'effet d'une précontrainte, avec la tige de poussoir, de façon que la tige de poussoir puisse se déplacer librement une première fois en direction de l'extrémité proximale, mais que le déplacement distal de la tige de poussoir ait pour effet d'appliquer la partie analogue à un burin contre la tige de poussoir afin que le dispositif de blocage se déplace avec la tige de poussoir en direction de l'extrémité distale, l'agencement étant tel que le dispositif de blocage (30, 32) permet de charger la seringue avec du médicament selon le volume défini par le dispositif de blocage par déplacement proximal du piston jusqu'à l'extrémité distale du dispositif de blocage et, après avoir poussé la tige de poussoir, d'expulser le médicament, tout second déplacement proximal de la tige de poussoir étant empêché par l'enfoncement de la partie en forme d'ergot dans la paroi latérale intérieure.

2. Une seringue à usage unique comprenant : un corps cylindrique creux allongé (2) possédant une paroi latérale intérieure, une extrémité proximale, une extrémité distale et une buse (8) fermant ladite extrémité distale dudit corps ;
un poussoir (10) disposé à l'intérieur dudit corps pour s'y déplacer axialement afin d'aspirer du fluide dans ledit corps et de l'en expulser à travers ladite buse, ledit élément formant poussoir comprenant une tige de poussoir, qui s'étend au-delà dudit corps au niveau de ladite extrémité proximale, et possédant un piston (16) à l'extrémité distale, un dispositif de blocage (30, 32) pouvant être disposé entre la tige de poussoir et la paroi intérieure, dans une position qui détermine le volume de la seringue que l'on veut remplir de médicament, caractérisée en ce que le dispositif de blocage est réalisé à partir de fil métallique plié qui présente la forme d'un U déformé et qui comprend une paire d'éléments formant bras (52) courbés vers le haut et vers l'extérieur, dont chacun est aiguisé pour former une pointe analogue à un burin (54), de façon que, lorsque le dispositif de blocage est en place entre la paroi latérale intérieure du corps cylindrique (2) et le poussoir (20), la tige de poussoir puisse se déplacer librement une première fois en direction de l'extrémité proximale, mais que le déplacement distal de la tige de poussoir ait pour effet d'appliquer la partie analogue à un burin contre la tige de poussoir afin que le dispositif de blocage se déplace avec la tige de poussoir en direction de l'extrémité distale, l'agencement étant tel que le dispositif de blocage (30, 32) permet de charger la seringue avec du médicament selon le volume défini par le dispositif de blocage par déplacement proximal du piston jusqu'à l'extrémité distale du dispositif de blocage et, après avoir poussé la tige de poussoir, d'expulser le médicament, tout second déplacement proximal de la tige de poussoir étant empêché par l'enfoncement de la partie en forme d'ergot dans la paroi latérale intérieure.

3. Une seringue conforme aux revendications 1 ou 2, dans laquelle ladite tige de poussoir comprend des pattes planes orientées longitudinalement (12) qui possèdent des surfaces pour y appliquer ladite partie analogue à un burin (36, 54).

4. Une seringue conforme à la revendication 1, dans laquelle ledit dispositif de blocage comprend une seconde partie en forme d'ergot (38) et une seconde partie analogue à un burin (34).

5. Une seringue conforme à la revendication 4, dans laquelle ladite partie analogue à un burin et ladite seconde partie analogue à un burin sont séparées par une fente longitudinale (40) pratiquée dans ledit élément, de sorte qu'à leur extrémité proximale, ladite partie analogue à un burin et ladite seconde partie analogue à un burin s'étendent à la façon de ressorts en porte-à-faux à partir de ladite partie distale dudit dispositif, ce qui a pour effet de pousser ladite partie analogue à un burin et ladite seconde partie analogue à un burin contre ladite paroi latérale intérieure dudit corps.

6. Une seringue conforme à la revendication 4, dans laquelle ladite tige de poussoir comprend au moins deux pattes planes (12) orientées longitudinalement qui possèdent des surfaces pour y appliquer ladite partie analogue à un burin (36) et ladite seconde partie (36) analogue à un burin, lesdites pattes (12) étant disposées de façon que lesdites parties analogue à un burin soient en contact avec des pattes planes différentes (12).

7. Une seringue conforme à la revendication 6, dans laquelle au moins deux desdites pattes planes (12) sont orientées de manière sensiblement perpendiculaire l'une à l'autre et forment ainsi, sur la longueur de ladite tige de poussoir, une rainure axiale en forme de V contre laquelle ledit dispositif de blocage peut être appliqué.

8. Une seringue conforme à l'une quelconque des revendications précédentes, dans laquelle ledit corps (2) est réalisé en matière plastique.

9. Une seringue conforme à l'une quelconque des revendications précédentes, dans laquelle ledit dispositif de blocage (30, 32) est réalisé en métal.

10. Une seringue conforme à la revendication 8, dans laquelle ledit dispositif de blocage (30, 32) est réalisé en tôle.

11. Une seringue conforme à la revendication 9, dans laquelle ladite tôle est en acier inoxydable.

12. Une seringue conforme à la revendication 1, dans laquelle ladite tige de poussoir possède une pluralité de pattes planes longitudinales (12) et dans laquelle ledit dispositif de blocage est placé uniquement entre des pattes planes adjacentes de ladite tige de poussoir.
